# EUROPEAN PATENT SPECIFICATION

(11) **EP 3 697 327 B1**
(45) Date of publication and mention of the grant of the patent: **11.05.2022**
(21) Application number: 18773970.1
(22) Date of filing: 17.09.2018
(51) Int. Cl.: A61B 17/3207, A61B 17/22, A61M 25/10

(54) **BALLOON CATHETER**
BALLONKATHETER
CATHÉTER À BALLONNET

(30) Priority: 18.10.2017 EP 17196972
(43) Date of publication of application: 26.08.2020
(73) Proprietor: Biotronik AG, 8180 Bülach (CH)
(72) Inventor: RISCH, Fabian, 8239 Dörflingen (CH); SCHÄFER, Tobias, 78176 Blumberg-Fützen (DE)
(74) Representative: Biotronik Corporate Services SE
(86) International application number: PCT/EP2018/075042
(87) International publication number: WO 2019/076558

(56) References cited:
- US-A- 4 762 130
- US-A1- 2002 045 852
- US-A1- 2004 143 287
- US-A1- 2013 060 127
- US-A1- 2013 237 909
- US-A1- 2014 324 079

## Description

The invention relates to a balloon catheter comprising at least a catheter shaft and an expandable balloon portion, and comprising working means for working on vascular deposits.

Balloon catheters and balloon catheter-stent devices have long been known and are used in a clinical setting in their hundreds of thousands for the treatment of vasoconstrictions (stenoses) of blood vessels. The long-known treatment by expansion (dilatation) by means of a balloon catheter is supplemented in devices of this kind by a stabilisation of the expanded vessel portion by means of a support element, that is to say the stent expanded by means of the balloon and left behind at the location of the stenosis following the deflation of the balloon and the withdrawal of the balloon catheter (stenting). Reference is made, purely by way of example, to DE 10 2004 059 523 A1 in respect of devices of this kind, said document describing a new stage of development of these devices.

In many cases an at least partial removal of deposits on the vessel wall is desirable in addition to the expansion and stabilisation of the stenosis portion. For this purpose, balloon catheters having what are known as cutting balloons with cutting blades on the balloon surface and more recently also stents having scoring elements for cutting vascular deposits have been developed and are also used already in a clinical setting.

Document WO 2009/046206 A1 presents a balloon catheter with attached scoring elements. Here, there are helically extending metal wires or comparable plastic elements attached to the balloon portion of the balloon catheter. Document US 8,348,987 B2 presents a balloon catheter-stent device with scoring elements attached to the stent structure.

It has been found that both solutions have significant disadvantages.

Cutting balloons are relatively rigid on account of the attached cutting elements and can only be introduced with difficulty into complex lesions, and there is a significant risk of perforation here as well. In addition, the minimum (deflated) balloon diameter is larger than in comparable balloons without cutting elements. Lastly, cutting balloons put up a relatively high resistance as the device is advanced through the vessel system, which hinders precise handling.

In stents with scoring elements there is the risk, *inter alia,* that the scoring elements, which also protrude in the insertion state, might cause tissue damage. On the other hand, the effects actually attained at the intended site of insertion have not proven to be satisfactory in clinical studies.

US 2013/237909 A1 discloses a balloon catheter with a catheter shaft and an expandable balloon portion, and with channels laid on the periphery of the balloon portion.

The object of the invention is therefore to provide an improved balloon catheter of the aforementioned type which can be handled easily and reliably and which can be configured in a versatile manner in accordance with different user requirements.

The invention is defined by claim 1. Further embodiments of the invention are defined by the dependent claims.

The invention includes the concept of equipping the balloon catheter with working means which in the starting state with deflated balloon, that is to say during the insertion of the balloon catheter, have a different physical characteristic than in the end state with inflated balloon, in which they are intended to be suitable for working on a stenosis. In particular, in the starting state they should have a low rigidity and should also protrude to the smallest possible extent beyond the peripheral surface of the (as yet uninflated) balloon portion, whereas in the end state they should protrude beyond the balloons surface to an extent sufficient for working and should be sufficiently rigid.

The invention also includes the concept of bringing about this "state transition" in a manner similar to that implemented in the case of the catheter balloon itself, specifically by filling with a suitable liquid. Lastly, the invention comprises the concept that the working means have at least one additional tube with a closed end, which tube is attached with a suitable profile to the balloon portion of the catheter and will be referred to hereinafter as an attachment tube.

Within the scope of this application, a balloon catheter is understood to mean a catheter which is suitable for being introduced into a bodily vessel or lumen of a patient, in particular into a lumen of the bloodstream. A balloon catheter of this kind comprises at least one catheter shaft and an expandable balloon portion, referred to as a balloon for short. The expansion (inflation) of the balloon is implemented by acting on said balloon with a fluid via a lumen of the catheter shaft.

In expedient embodiments of the invention a plurality of attachment tubes of this kind, in particular 3 to 5 attachment tubes, is provided. Improved working effects can be achieved hereby compared with an individual attachment tube.

In particular, the attachment tube or the attachment tubes lies/lie helically on the balloon portion, wherein it/they describes/describe in particular at least a half turn about the longitudinal axis of the balloon portion between the proximal balloon neck and the distal balloon neck. The angular range of a helical course between proximal and distal balloon neck of the attachment tube or the attachment tubes will be determined by a person skilled in the art in accordance with the specific configuration, in particular in respect of the length of the balloon portion, the number of attached tubes, and requirements of the specific application. In particular if a plurality of attachment tubes are provided, it is also possible in principle to lay said attachment tubes parallel to the longitudinal axis of the balloon portion.

In an example not according to the invention, the attachment tube or each attachment tube is welded or glued at least over the majority of its length to the balloon portion. In accordance with the present claimed invention, the attachment tube is fixedly connected, in particular welded or adhesively bonded, to the catheter shaft substantially only at the proximal balloon neck. In the latter variant it would appear to be advantageous if the attachment tube or each attachment tube is also welded or glued therebetween at specific points to the balloon portion so as to prevent undesirable tangential and/or axial displacements of the tube or the tubes relative to the balloon and associated deteriorations of the working effect.

In a further embodiment the catheter shaft comprises an inner shaft and an outer shaft, and the open proximal end of the attachment tube or each attachment tube is connected fluidically to the lumen of the outer shaft. The attachment tube or the attachment tubes is/are pressurised at this moment, that is to say simultaneously with the catheter balloon, which makes the structure of the catheter as a whole and also handling thereof particularly simple. In another catheter construction known per se, the catheter shaft has a first lumen for a guide wire and a second lumen for introducing a fluid for expanding the balloon portion, and the open proximal end of the attachment tube or each attachment tube is fluidically connected to the second lumen.

Alternatively, it is provided that the open proximal end of the attachment tube or each attachment tube opens out into a separate proximal fluid connection of the balloon catheter. This offers the possibility of controlling the characteristic of the working means independently of the inflation of the balloon, but requires a separate fluid line extending as far as the proximal catheter end or an additional lumen in the catheter body and a separate connection of the catheter.

Here, it is possible on the one hand that the open proximal end of the attachment tube or each attachment tube is connected via an additional lumen in the catheter shaft to the dedicated fluid connection. Alternatively, as a modification of known catheter configurations, the proximal end of the attachment tube or each outer tube can be connected to the separate fluid connection via a working fluid tube laid externally on the catheter shaft.

In a further embodiment of the invention the balloon catheter has a working means protective sleeve displaceable along the catheter shaft. This protective sleeve is advanced as far as the distal balloon neck before and during insertion of the catheter and covers the outer tube or the outer tubes, and is withdrawn as far as at least the proximal balloon neck after the insertion, so as to expose the outer tube or the outer tubes for implementation of its/their working purpose. It goes without saying that in a construction of this kind an additional actuation element is required for the working means protective sleeve, and the handling of the catheter of this kind is slightly more complex.

In expedient embodiments the attachment tube or each attachment tube comprises a material from the group of polyamides, polyimides, polyesters, polyethers, polyolefins and copolymers thereof. Materials which are compatible with the material of the catheter balloon such that light welding or adhesive bonding is possible are particularly preferred.

In further embodiments of the invention the diameter of the attachment tube or each attachment tube lies in the range between 2% and 8%, preferably between 2% and 4%, of the diameter of the balloon. The specific determination of the attachment tube geometry will be made by a person skilled in the art as a matter of routine, in particular under consideration of the structure of the balloon catheter per se and the specific situation of application.

In further embodiments it is provided that the attachment tube or at least part of the attachment tubes comprises a cutting or sawing edge for abrasive working on vascular deposits. The cutting or sawing edge(s) can be adhesively bonded or welded to the attachment tube proximally, distally and/or over the entire length. Alternatively, the cutting or sawing edge(s) can also be produced directly with the forming of the attachment tube. Materials that are suitable for the cutting or sawing edge are in particular metals (for example stainless steel) or polymers, such as polyamides, polyimides, polyesters, polyethers, polyolefins and copolymers thereof. The diameter of an attachment tube (or maximum radial extent thereof) is advantageously between 4% and 8% of the balloon diameter. This is advantageous in particular for applications in which the balloon catheter should not only break through calcareous deposits, but should also separate and/or remove them in layers. It goes without saying that the provision of cutting or sawing edges can slightly hamper the handling of the balloon catheter during the insertion process; this disadvantage, however, is outweighed in certain application situations by the advantages of an embodiment of this kind.

The following advantages, inter alia, can be attained with the invention at least in certain embodiments:
Advantages and expedient features of the invention will also become clear from the following description of an exemplary embodiment with reference to the drawings, in which:
- Fig. 1: shows a schematic perspective view of a balloon catheter in accordance with a first embodiment of the invention,
- Fig. 2: shows a schematic perspective view of a balloon catheter in accordance with a further embodiment of the invention,
- Fig. 3: shows a schematic perspective view of a balloon catheter in accordance with a further embodiment of the invention,
- Fig. 4: shows a schematic longitudinal sectional view of the main structure of a balloon catheter,
- Fig. 5: shows a schematic longitudinal sectional view of a second main structure of a balloon catheter,
- Fig. 6: shows a schematic longitudinal sectional view of a balloon catheter in accordance with a further embodiment of the disclosure.

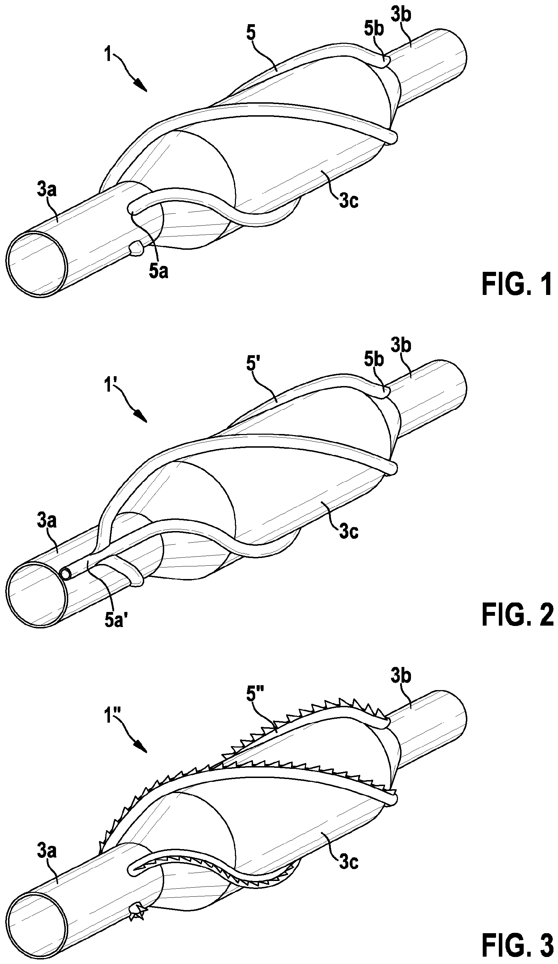

Fig. 1 shows a sketch of a balloon catheter 1 which comprises an outer shaft 3a, an inner shaft 3b, and a balloon portion 3c (shown in the inflated state in the drawing), and also three attachment tubes 5 laid on the balloon portion 3c. The attachment tubes 5 laid helically at equal angular distances over the peripheral surface of the balloon portion 3c each have a proximal open end. They are connected there fluidically at mouth points 5a to the inner lumen of the catheter shaft 3a, that is to say are subjected to fluid pressure simultaneously with the inflation of the balloon 3c and in the same way as the balloon portion. At their distal ends 5b, the attachment tubes 5 are closed in a fluid-tight manner. The attachment tubes can be welded, adhesively bonded or otherwise fixedly connected only at specific points, for example at the neck portions of the balloon 3c, to the balloon 3c and/or the adjoining shaft portions.

Fig. 2 shows a modification of the structure shown in Fig. 1, wherein the same reference numerals are provided for matching or functionally similar parts, and those parts will not be described further here. The main difference of the balloon catheter 1' from the balloon catheter 1 according to Fig. 2 lies in a modified embodiment of the attachment tubes 5' at the proximal ends thereof. Here, these attachment tubes specifically do not open out into the catheter shaft 3a, but instead are connected to one another via a branch piece 5a' and are connected to a separate fluid line (not shown) leading to the proximal catheter end. In this embodiment the attachment tubes 5' are placed under fluid pressure via the separate fluid line, independently of the inflation of the balloon portion 3c.

Fig. 3 shows a further balloon catheter 1", which is derived from the balloon catheter 1 according to Fig. 1 by way of a modification. The main difference here lies in the fact that a sawtooth structure is formed on the attachment tubes 5" and enables abrasive working on vascular deposits at the site of use of the balloon catheter.

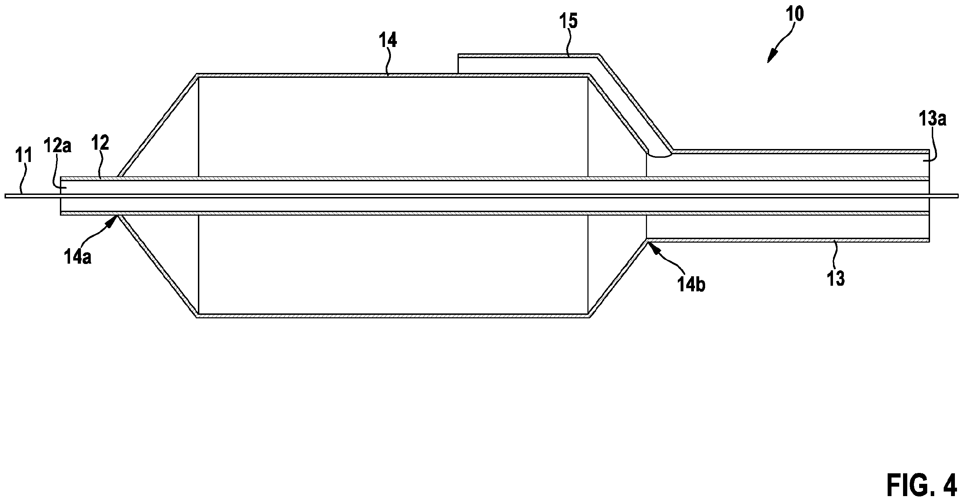

Fig. 4 schematically shows, in the form of a longitudinal sectional view, a first variant of the inner structure of a balloon catheter 10 with a substantially coaxial structure. The catheter shaft (not referenced separately here) comprises an inner shaft 12, in which a guide wire 11 is received, and an outer shaft 13. A balloon portion 14 is welded to the inner shaft 12 at a distal balloon neck 14a and to the outer shaft 13 at a proximal balloon neck 14b. The balloon portion 14 is shown here in the expanded state. In order to produce this state, a lumen 13a for feeding a fluid into the balloon portion 14 is provided in the outer shaft 13, whereas an inner shaft lumen 12a coaxial with the lumen 13a receives the guide wire 11 longitudinally displaceably in the inner shaft 12. An attachment tube 15 as working means of the balloon catheter 10 is fluidically connected at the proximal balloon neck 14b to the outer shaft lumen 13a. The attachment tube 15 is thus inflated simultaneously with the balloon portion 14.

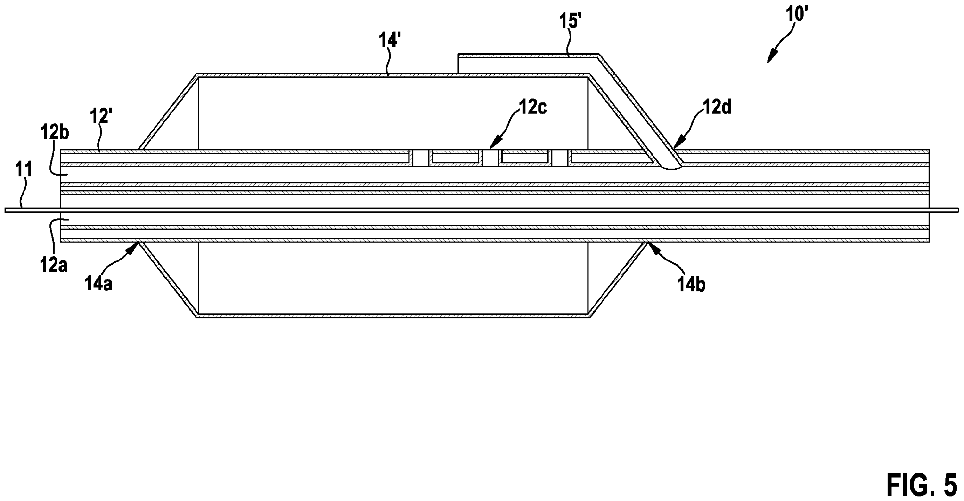

Fig. 5 shows a further balloon catheter 10' with a different structure, wherein like or functionally comparable parts are denoted by the same numbers as in Fig. 4 and will not be described here again. In contrast to the balloon catheter 10 according to Fig. 4, the balloon catheter 10' has a one-piece catheter shaft 12', in which two lumens 12a (for the guide wire 11) and 12b (as fluid channel) are incorporated parallel to one another. The catheter shaft 12' is welded to the balloon portion 14' both at the distal balloon neck 14a and at the proximal balloon neck 14b. The balloon portion 14' is filled via passages 12c from the fluid channel 12b into the interior of the balloon portion 14' with a fluid fed from the proximal catheter and is expanded hereby. A further passage 12d connects the fluid channel 12b to the attachment tube 15'. Fluid in the lumen 12b thus again places the attachment tube under fluid pressure simultaneously with the balloon portion 14' and expands said tube. Alternatively to the shown configuration, the attachment tube 15' can also run externally on the catheter shaft 12' to the proximal catheter end.

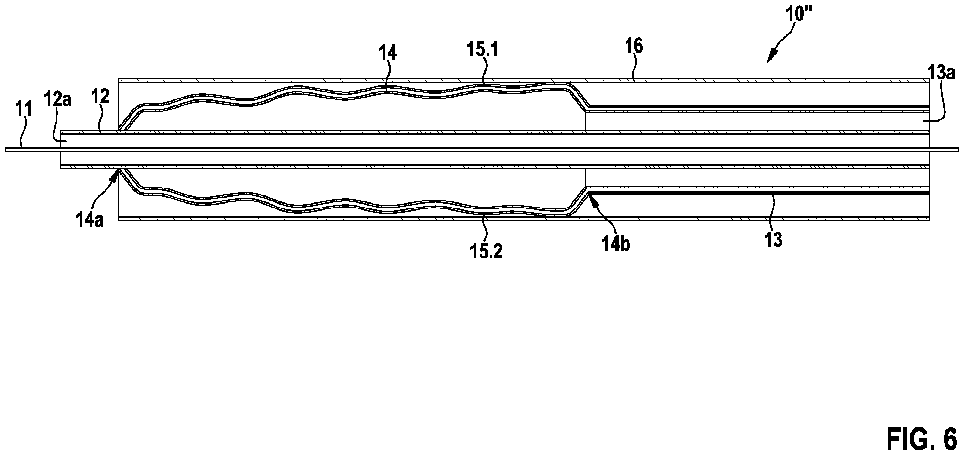

A further alternative to the configurations shown in Fig. 4 and Fig. 5 lies in that an additional lumen is provided within the outer catheter in the arrangement according to Fig. 4 or within the catheter shaft in the arrangement according to Fig. 5, which additional lumen is used exclusively to supply fluid to the attachment tube or the attachment tubes. This variant is shown schematically in Fig. 6, more specifically together with a further modification of the configuration according to Fig. 4. This lies in that a working means protective sleeve 16 is associated with the actual balloon catheter C2'. This protective sleeve covers the attachment tubes 15.1 and 15.2 (and any further attachment tubes; not shown here) in the insertion state and is withdrawn proximally after insertion, when the balloon catheter is placed in operation with its attachment tubes serving as working means.

Otherwise, the invention can also be embodied in a large number of modifications of the examples shown here and aspects of the invention described further above.

## Claims

1. A balloon catheter (1; 1', 1"; 10; 10'; 10") with a catheter shaft (12'; 12/13) and an expandable balloon portion (3c; 14; 14'), and with a working means for working on vascular deposits,
**characterised in that** the working means comprises at least one proximally open and distally closed attachment tube (5; 5', 5"; 15; 15'; 15.1, 15.2) or a plurality of attachment tubes (5; 5', 5"; 15; 15'; 15.1, 15.2), in particular three to five attachment tubes, laid on the periphery of the balloon portion, and wherein the attachment tube or each attachment tube (5; 5', 5"; 15; 15'; 15.1, 15.2) is securely fixed to the catheter shaft (12'; 12/13) substantially only at the proximal balloon neck (14b) and at the distal balloon neck (14a).

2. The balloon catheter according to claim 1, wherein the attachment tube or the attachment tubes (5; 5', 5"; 15; 15'; 15.1, 15.2) is/are laid helically on the balloon portion (3c, 14; 14'), wherein it/they describes/describe in particular at least one half turn about the longitudinal axis of the balloon portion between the proximal balloon neck (14b) and the distal balloon neck (14a).

3. The balloon catheter according to claim 1 or 2, wherein the attachment tube or each attachment tube (5; 5', 5"; 15; 15'; 15.1, 15.2) is welded or adhesively bonded to the catheter shaft (12'; 12/13) substantially only at the proximal balloon neck (14b) and at the distal balloon neck (14a)

4. The balloon catheter according to claim 1 or 2, wherein the attachment tube or each attachment tube (5; 5', 5"; 15; 15'; 15.1, 15.2) is welded or glued at specific points to the balloon portion (3c; 14, 14') between the proximal and distal balloon neck (14b, 14a).

5. The balloon catheter according to any one of the preceding claims, wherein the catheter shaft comprises an inner shaft (3b; 12) and an outer shaft (3a; 13), and the open proximal end (5a) of the attachment tube or each attachment tube (5; 15) is fluidically connected to the lumen of the outer catheter (3a; 13).

6. The balloon catheter according to any one of claims 1 to 4, wherein the catheter shaft (12'; 12/13) comprises a first lumen (12a) for a guide wire (11) and a second lumen (12b'; 13a) for introducing a fluid for expanding the balloon portion, and the open proximal end of the attachment tube or each attachment tube is fluidically connected to the second lumen (12b; 13a).

7. The balloon catheter according to any one of claims 1 to 4, wherein the open proximal end (5a') of the attachment tube or each attachment tube (5'; 15.1, 15.2) opens out into a dedicated proximal fluid connection of the balloon catheter (1'; 10").

8. The balloon catheter according to claim 7, wherein the open proximal end of the attachment tube or each attachment tube is connected to the dedicated fluid connection via an additional lumen in the catheter shaft.

9. The balloon catheter according to claim 7, wherein the open proximal end of the attachment tube or each attachment tube (15.1, 15.2) is connected to the dedicated fluid connection via a working fluid tube laid externally on the catheter shaft (12/13).

10. The balloon catheter according to any one of the preceding claims, **characterised in that** further having a working means protective sleeve (16) displaceable along the catheter shaft (12/13).

11. The balloon catheter according to any one of the preceding claims, wherein the attachment tube or each attachment tube (5; 5', 5"; 15, 15'; 15.1, 15.2) comprising a material from the group of polyamides, polyimides, polyesters, polyethers, polyolefins and copolymers thereof.

12. The balloon catheter according to any one of the preceding claims, wherein the diameter of the attachment tube or each attachment tube (5; 5', 5"; 15, 15'; 15.1, 15.2) lies in the range between 2% and 8%, preferably between 2% and 4%, of the diameter of the balloon.

13. The balloon catheter (1") according to any one of the preceding claims, wherein the attachment tube or at least part of the attachment tubes (5") has a cutting or sawing edge for abrasive working on vascular deposits.

## Patentansprüche

1. Ballonkatheter (1; 1', 1"; 10; 10'; 10") mit einem Katheterschaft (12'; 12/13) und einem expandierbaren Ballonteil (3c; 14; 14'), und mit einem Arbeitsmittel zur Bearbeitung von Gefäßablagerungen,
**dadurch gekennzeichnet, dass** das Arbeitsmittel mindestens ein proximal offenes und distal geschlossenes Aufsatzrohr (5; 5', 5"; 15; 15'; 15.1, 15.2) oder mehrere Aufsatzrohre (5; 5', 5"; 15; 15'; 15.1, 15. 2), insbesondere drei bis fünf Aufsatzrohr, die an der Peripherie des Ballonabschnitts verlegt sind, und wobei das oder jedes Aufsatzrohr (5; 5', 5"; 15; 15'; 15.1, 15.2) im Wesentlichen nur am proximalen Ballonhals (14b) und am distalen Ballonhals (14a) fest mit dem Katheterschaft (12'; 12/13) verbunden ist.

2. Ballonkatheter nach Anspruch 1, wobei das Aufsatzrohr oder die Aufsatzrohre (5; 5', 5"; 15; 15'; 15.1, 15.2) schraubenförmig auf den Ballonabschnitt (3c, 14; 14') gelegt ist/sind, wobei es/sie insbesondere mindestens eine halbe Drehung um die Längsachse des Ballonabschnitts zwischen dem proximalen Ballonhals (14b) und dem distalen Ballonhals (14a) beschreibt/beschreiben.

3. Ballonkatheter nach Anspruch 1 oder 2, wobei das Aufsatzrohr oder jedes Aufsatzrohr (5; 5', 5"; 15; 15'; 15.1, 15.2) im Wesentlichen nur am proximalen Ballonhals (14b) und am distalen Ballonhals (14a) mit dem Katheterschaft (12'; 12/13) verschweißt oder verklebt ist.

4. Ballonkatheter nach Anspruch 1 oder 2, **dadurch gekennzeichnet, dass** das Aufsatzrohr oder jedes Aufsatzrohr (5; 5', 5"; 15; 15'; 15.1, 15.2) an bestimmten Stellen mit dem Ballonteil (3c; 14, 14') zwischen dem proximalen und distalen Ballonhals (14b, 14a) verschweißt oder verklebt ist.

5. Ballonkatheter nach einem der vorhergehenden Ansprüche, wobei der Katheterschaft einen inneren Schaft (3b; 12) und einen äußeren Schaft (3a; 13) umfasst und das offene proximale Ende (5a) des oder jedes Aufsatzrohrs (5; 15) fluidisch mit dem Lumen des äußeren Katheters (3a; 13) verbunden ist.

6. Ballonkatheter nach einem der Ansprüche 1 bis 4, wobei der Katheterschaft (12'; 12/13) ein erstes Lumen (12a) für einen Führungsdraht (11) und ein zweites Lumen (12b'; 13a) zum Einleiten eines Fluids zur Aufweitung des Ballonabschnitts aufweist und das offene proximale Ende des Aufsatzrohrs oder jedes Aufsatzrohrs mit dem zweiten Lumen (12b; 13a) fluidisch verbunden ist.

7. Ballonkatheter nach einem der Ansprüche 1 bis 4, wobei das offene proximale Ende (5a') des Aufsatzrohrs oder jedes Aufsatzrohrs (5'; 15.1, 15.2) in einen zugehörigen proximalen Fluidanschluss des Ballonkatheters (1'; 10") mündet.

8. Ballonkatheter nach Anspruch 7, bei dem das offene proximale Ende des Aufsatzrohrs oder jedes Aufsatzrohrs über ein zusätzliches Lumen im Katheterschaft mit dem zugehörigen Fluidanschluss verbunden ist.

9. Ballonkatheter nach Anspruch 7, **dadurch gekennzeichnet, dass** das offene proximale Ende des Aufsatzrohrs oder jedes Aufsatzrohrs (15.1, 15.2) über einen außen am Katheterschaft (12/13) verlegten Arbeitsflüssigkeitsschlauch mit dem zugehörigen Fluidanschluss verbunden ist.

10. Ballonkatheter nach einem der vorhergehenden Ansprüche, **dadurch gekennzeichnet, dass** er ferner eine Arbeitsmittelschutzhülse (16) aufweist, die entlang des Katheterschafts (12/13) verschiebbar ist.

11. Ballonkatheter nach einem der vorhergehenden Ansprüche, wobei das Aufsatzrohr oder jedes Aufsatzrohr (5; 5', 5"; 15, 15'; 15.1, 15.2) aus einem Material aus der Gruppe der Polyamide, Polyimide, Polyester, Polyether, Polyolefine und deren Copolymere besteht.

12. Ballonkatheter nach einem der vorhergehenden Ansprüche, wobei der Durchmesser des Aufsatzrohrs oder jedes Aufsatzrohrs (5; 5', 5"; 15, 15'; 15.1, 15.2) im Bereich zwischen 2% und 8%, vorzugsweise zwischen 2% und 4%, des Ballondurchmessers liegt.

13. Ballonkatheter (1") nach einem der vorhergehenden Ansprüche, wobei das Aufsatzrohr oder zumindest ein Teil der Aufsatzrohre (5") eine Schneid- oder Sägekante zur abrasiven Bearbeitung von Gefäßablagerungen aufweist.

## Revendications

1. Cathéter à ballonnet (1; 1', 1'; 10; 10'; 10") avec une tige de cathéter (12'; 12/13) et une partie de ballonnet expansible (3c; 14; 14'), et avec un moyen de travail pour travailler sur des dépôts vasculaires,
**caractérisé en ce que** le moyen de travail ouvert proximalement et fermé distalement (5; 5', 5"; 15; 15'; 15.1, 15.2) ou une pluralité de tubes d'attache (5; 5, 5"; 15; 15'; 15.1, 15. 2), en particulier trois à cinq tubes d'attache, posés sur la périphérie de la partie de ballon, et dans lequel le tube d'attache ou chaque tube d'attache (5; 5', 5"; 15; 15'; 15.1, 15.2) est fixé solidement à la tige de cathéter (12'; 12/13) sensiblement seulement au niveau du col de ballon proximal (14b) et du col de ballon distal (14a).

2. Cathéter à ballonnet selon la revendication 1, dans lequel le tube d'attache ou les tubes d'attache (5; 5', 5"; 15; 15'; 15.1, 15.2) est/sont posé(s) en hélice sur la partie de ballonnet (3c, 14; 14'), dans lequel il/ils décrit(s) en particulier au moins un demi-tour autour de l'axe longitudinal de la partie de ballonnet entre le col de ballonnet proximal (14b) et le col de ballonnet distal (14a).

3. Cathéter à ballonnet selon la revendication 1 ou 2, dans lequel le tube d'attache ou chaque tube d'attache (5; 5', 5"; 15; 15'; 15.1, 15.2) est soudé ou collé à la tige du cathéter (12'; 12/13) sensiblement seulement au niveau du col de ballonnet proximal (14b) et du col de ballonnet distal (14a).

4. Cathéter à ballonnet selon la revendication 1 ou 2, dans lequel le tube d'attache ou chaque tube d'attache (5; 5', 5"; 15; 15'; 15.1, 15.2) est soudé ou collé en des points spécifiques à la partie de ballonnet (3c; 14, 14') entre le col de ballonnet proximal et distal (14b, 14a).

5. Cathéter à ballonnet selon l'une quelconque des revendications précédentes, dans lequel la tige du cathéter comprend une tige intérieure (3b; 12) et une tige extérieure (3a; 13), et l'extrémité proximale ouverte (5a) du tube d'attache ou de chaque tube d'attache (5 ; 15) est reliée fluidiquement à la lumière du cathéter extérieur (3a ; 13).

6. Cathéter à ballonnet selon l'une quelconque des revendications 1 à 4, dans lequel la tige de cathéter (12'; 12/13) comprend une première lumière (12a) pour un fil de guidage (11) et une deuxième lumière (12b'; 13a) pour introduire un fluide pour dilater la partie ballonnet, et l'extrémité proximale ouverte du tube d'attache ou de chaque tube d'attache est reliée fluidiquement à la deuxième lumière (12b; 13a).

7. Cathéter à ballonnet selon l'une quelconque des revendications 1 à 4, dans lequel l'extrémité proximale ouverte (5a') du tube d'attache ou de chaque tube d'attache (5'; 15.1, 15.2) débouche dans une connexion fluidique proximale dédiée du cathéter à ballonnet (1'; 10").

8. Cathéter à ballonnet selon la revendication 7, dans lequel l'extrémité proximale ouverte du tube d'attache ou de chaque tube d'attache est reliée à la connexion fluidique dédiée via une lumière supplémentaire dans la tige du cathéter.

9. Cathéter à ballonnet selon la revendication 7, dans lequel l'extrémité proximale ouverte du tube d'attache ou de chaque tube d'attache (15.1, 15.2) est connectée à la connexion fluidique dédiée via un tube fluidique de travail posé à l'extérieur sur la tige du cathéter (12/13).

10. Le cathéter à ballonnet selon l'une quelconque des revendications précédentes, **caractérisé en ce qu'**il comporte en outre un manchon de protection du moyen de travail (16) déplaçable le long de la tige du cathéter (12/13).

11. Cathéter à ballonnet selon l'une quelconque des revendications précédentes, dans lequel le tube d'attache ou chaque tube d'attache (5; 5', 5"; 15, 15'; 15.1, 15.2) comprend un matériau du groupe des polyamides, polyimides, polyesters, polyéthers, polyoléfines et leurs copolymères.

12. Cathéter à ballonnet selon l'une quelconque des revendications précédentes, dans lequel le diamètre du tube d'attache ou de chaque tube d'attache (5; 5', 5"; 15, 15'; 15.1, 15.2) est compris entre 2% et 8%, de préférence entre 2% et 4%, du diamètre du ballonnet.

13. Cathéter à ballonnet (1") selon l'une quelconque des revendications précédentes, dans lequel le tube d'attache ou au moins une partie des tubes d'attache (5") présente un bord coupant ou sciant pour un travail abrasif sur les dépôts vasculaires.
